Europäisches Patentamt

European Patent Office

Office européen des brevets

(11) Publication number: **0 222 725 B1**

# EUROPEAN PATENT SPECIFICATION

(45) Date of publication of patent specification: **11.03.92**

(51) Int. Cl.⁵: **C12N 1/06**, C12N 9/36, C12P 21/02

(21) Application number: **86870159.0**

(22) Date of filing: **28.10.86**

(54) **Process for recovering polypeptides localized in the periplasmic space of yeast.**

(30) Priority: **13.11.85 BE 215849**

(43) Date of publication of application:
**20.05.87 Bulletin 87/21**

(45) Publication of the grant of the patent:
**11.03.92 Bulletin 92/11**

(84) Designated Contracting States:
**AT BE CH DE ES FR GB GR IT LI LU NL SE**

(56) References cited:
**WO-A-87/01128**
**BE-A- 903 626**

**FOOD TECHNOLOGY, vol. 28, no. 10, October 1974, Chicago, US; H. SUGIMOTO: "Treatment of soybean spent solubles by means of yeast cultivation" & "synergistic effect of ethanol and sodium chloride on autolysis of baker's yeast for preparing food-grade yeast extracts"**

(73) Proprietor: **FINA RESEARCH S.A.**
**Zone Industrielle C**
**B-7181 Feluy(BE)**

(72) Inventor: **De Baetselier-van Broekoven, Annie J.F.**
**Mevrouw Courtmanstraat, 9**
**B-2600 Berchem(BE)**
Inventor: **Crahay, Jacques**
**51, Ferme d'Hermoye**
**B-5830 Mazy(BE)**
Inventor: **Delcour, Jean M.A.G.**
**rue des Boscailles, 2**
**B-5865 Walhain(BE)**
Inventor: **Hanotier, Jacques D.V.**
**Rue de Caturia, 5b**
**B-1338 Lasne(BE)**

(74) Representative: **Leyder, Francis**
**c/o Fina Research S.A. Zone Industrielle C**
**B-7181 Feluy(BE)**

**Description**

The present invention relates to a process for releasing into aqueous medium polypeptides produced by yeasts and localized at least partially in the periplasmic space thereof. More particularly, it relates to a process for releasing into aqueous medium the lysozyme produced by yeasts genetically engineered for that purpose.

The yeast Saccharomyces cerevisiae is increasingly used as host for genetic manipulations aiming at producing by fermentation polypeptides of commercial interest. This increasing use of yeast can be explained by the various advantages that it shows over other industrial microorganisms, e.g. the fact that yeast is above all an alimentary organism. Another advantage of yeast is that its culture does not require absolute sterile conditions, so that it is particularly appropriate for large scale fermentations. Its ability to live under anaerobic conditions makes it also suitable in immobilized form for the continuous production of metabolites.

When using yeast for producing polypeptides, e.g. enzymes, it is obviously important that these be secreted through the plasmic membrane and excreted into the fermentation medium, from which they can then be recovered by using well known techniques such as adsorption or affinity chromatography. It is known that proteins secreted through the plasmic membrane of yeast tend to remain confined to the periplasmic space or at least to remain associated to the cell wall. This is often observed in yeasts of the Saccharomyces genus and particularly in the S. cerevisiae species (R. SCHEKMAN and P. NOVICK, the Molecular Biology of Yeast Saccharomyces, Metabolism and Gene Expression, J.N. Strathern et al. Eds, Cold Spring Harbor, New York, 1982, pp 361-393). This peculiarity which may bring some functional advantage to yeast is however a major disadvantage from a practical standpoint when the production by fermentation of proteins of commercial interest is to be carried out. Indeed, in such a case, the benefit brought by secretion for recovering the protein of interest is lost since said protein, by remaining associated to the cells, must be separated from the whole of the cellular materials as in the case of an intracellular protein. This tendency of yeasts for keeping associated to their wall the proteins they secrete has been attributed to the fact that most of these proteins are heavily glycosylated. This is the case for invertase and for acid phosphatase which both contain a large proportion of polysaccharides comprising essentially mannose. One function of the glycosylated portion of these proteins would be to maintain them associated to the polysaccharide matrix of the wall comprising itself essentially mannose (J.O. LAMPEN, Antonie van Leeuwenhoek, 34, 1-18, 1968). According to this interpretation, the excretion into the medium of the $\alpha$-factor of Mat$\alpha$ - type yeasts or of the "killer" protein produced by some yeast strains can be explained by the fact that they are not glycosylated.

However, when non-glycosylated heterologous proteins are expressed in yeast, it often happens that a fraction only of the protein formed is excreted into the medium, even when it is equipped with a signal sequence allowing its secretion through the plasmic membrane. This is the case for human $\alpha$1 interferon (A. SINGH et al., Nucleic Acids Res., 12, 8927-8938, 1984) the secretion of which was ensured by fusion of the corresponding gene with the DNA coding for the leader sequence of the precursor of the yeast $\alpha$ factor : only one half thereof is found in the medium. This is also the case of chicken lyzozyme secreted thanks to its own signal sequence (Belgian patent n° 901,223). In such cases, it is obviously possible to recover only the soluble fraction of the protein but this would result in a loss of yield. It is also possible to recover the fraction remaining associated to the cell, but this would require additional operations. Either solution would result in increased production costs which would reduce to some extent the hereabove enumerated advantages of using yeast as a production organism.

The main object of the present invention is to provide a process for recovering in a simple way and in high yield the polypeptides produced and secreted by yeast but which remain partially or totally localized within the periplasmic space. More specifically, the object of the present invention is to recover in high yield the heterologous proteins produced and excreted at least partially into the medium by yeasts genetically engineered for that purpose. Still more specifically, the object of the present invention is to recover from the yeast S. cerevisiae the lysozyme produced therein and secreted as a result of a genetic manipulation.

According to the present invention, these objects are achieved by a process consisting of treating these yeasts in aqueous medium by a system comprising (i) a neutral soluble mineral salt and (ii) a soluble nonionic surfactant of the polyethoxylated alkylphenol type, the HLB (Hydrophilic Lipophilic Balance) of which is comprised between 8 and 15.

It is known that the presence in sufficient concentration of sodium chloride or of other salts such as KCl or NaNO$_3$ in the culture medium of yeast favours the liberation therein of soluble proteins. Thus, it was shown that in the presence of 0.6 M NaCl, the amount of proteins in the medium after 6 hours culture of a bakers' yeast in an aerated medium was three times higher than in the absence of salt (T.A. FRANKLIN et al., Biotechnology and Bioengineering Symp. n° 14, 467, 1984). This result cannot be explained by a liberation of proteins as a result of an osmotic shock, as it is admitted that the periplasmic proteins of S. cerevisiae are not liberated by this treatment (W.N. ARNOLD, Physical Aspects of the Yeast Cell Envelope; in Yeast Cell Envelopes : Biochemistry, Biophysics and Ultrastructure vol. 1, Ed. W.N. ARNOLD, CRC Press Inc, Boca Raton, Florida, 1981, p. 25-47). Although the mechanism of action of the salt is not precisely known, it may be supposed that ionic bonds can take place between the yeast cell wall and proteins soluble in the medium, the effect of the salt being to break these bonds and to liberate the proteins. This phenomenon can also be evidenced by the following experiment.

Yeasts (S. cerevisiae, GRF18 strain) grown in a minimal medium (3% glucose, 0.67% yeast nitrogen base, 0.002% histidine and leucine) and harvested at the beginning of the stationary phase are resuspended in a 0.1 M phosphate buffer pH 6.5 to which chicken lysozyme (Boehringer Ingelheim) was added up to a concentration of 670 units/ml suspension. After 2 hours incubation at 37°C, the cells are separated by centrifugation and the lysozyme present in the supernatant is determined by the method of D. SHUGAR (Biochem. Biophys. Acta 8, 302-309, 1952). It is thus shown that the lysozyme concentration is only 440 units/ml, i.e. 66% of the initial value. The same result is observed at 0°C. This cannot be attributed to a partial deactivation of the enzyme during incubation, because lysozyme incubated at the same concentration and at the same temperatures in the supernatant of the initial culture does not show any loss of activity.

In another experiment, after one hour incubation at 4°C in the presence of lysozyme, NaCl was added up to a concentration of 0.5 M to the yeast suspension which was further incubated at 30°C during 2 hours. The suspension was then centrifuged and the lysozyme present in the supernatant was determined as hereabove. It was thus determined that 98% of the initial lysozyme is in solution in the supernatant, as opposed to 38% when NaCl is omitted in another identical experiment.

These experiments tend to show that extraneous lysozyme binds reversibly to the yeast cell wall, wherefrom it can be released quantitatively by increasing the ionic strength of the medium. The situation is not the same when lysozyme associated to the cells results from the expression therein of a cloned gene. It is known (Belgian Patent 901,223) that it is possible to clone and express in yeast foreign genes coding for enzymes having a 1,4-$\beta$-N-acetylmuramidasic activity, e.g. the gene coding for chicken lysozyme. The lysozyme thus formed in yeast shows such an activity; it is partially present in the culture medium, from which it was separated by adsorption on fast flow carboxymethylsepharose (Pharmacia) in 0.1 M phosphate buffer pH 6.5, followed by washing with the same buffer then by elution in the same buffer supplemented with 0.5 M NaCl. The eluate thus obtained was then concentrated by ultrafiltration, desalted by passage over Sephadex G-25 (Pharmacia), reconcentrated by ultrafiltration and dried by lyophilisation. Electrophoresis on polyacrylamide gel in the presence of sodium dodecylsulfate showed only one protein band, with an electrophoretic mobility identical to that of commercial chicken lysozyme extracted from egg white.

As a matter of fact, the sequence of the first ten amino acids of the N-terminal end of the protein extracted from the culture medium by the hereabove described method was shown to be identical to that of mature chicken lysozyme. This result clearly demonstrates that the signal peptide of the prelysozyme formed in yeast by expression of the cloned gene is correctly recognized and processed by the yeast, so that lysozyme is secreted by the yeast in active form. However, as will be evidenced by the following examples, a fraction of the lysozyme produced by the yeast is not liberated in the medium even when, as suggested by the hereabove described experiment, the cells are incubated in the presence of 0.5 M NaCl. This lysozyme fraction can be detected after grinding the cells and centrifuging the cell debris, especially when grinding is carried out in the presence of 0.5 M NaCl. It can thus be concluded that said lysozyme corresponds to a soluble fraction (intracellular or periplasmic), even if, as the effect of NaCl seems to indicate, it can partly be adsorbed on one or another cell constituant.

However, the possibility exists that a non negligible portion of the lysozyme produced by the yeast be associated to membrane structures (plasmic membrane and/or intracellular structures), possibly in the form of prelysozyme. It is known for example that calf chymosine expressed in yeast remains for a good part associated after lysis of the cells to centrifugable cell debris, even after thorough washing thereof (J. MELLOR et al., Gene 24, 1-14, 1983). In some cases, the proteins associated with membrane structures can be dissolved by treatment with a mild surfactant of the ethoxylated alkylphenol type (A. HELENIUS and K. SIMONS, Biochem. and Biophys. Acta, 415, 29-79, 1975). This treatment sometimes allows to release proteins associated to the cells. Thus, by treating a suspension of Saccharomycopsis lipolytica with an ethoxylated nonylphenol (Emulgen 950, Kao Atlas Co), the lipase associated therewith can be dissolved without lysis of the cells (Y. OTA et al., Agric. Biol. Chem., 46, 2885-2893, 1982). However, when the same procedure is applied to S. cerevisiae genetically manipulated to produce lysozyme, no release of this can be observed. Even when the yeast cells producing lysozyme are grinded in the presence of a polyethoxylated octylphenol such as Triton X-100 (Rohm and Haas) at a concentration of 0.05%, the lysozyme activity determined in the supernatant is not significantly different from that observed in the absence of the surfactant.

These various results show that, in order to recover the major part of the lysozyme produced by yeasts genetically manipulated for that purpose, it is not sufficient to treat the cells with a soluble salt, nor even with a surfactant as suggested by the prior art; it is still necessary, when resorting to known methods, to destroy the cell structure, with all the difficulties brought by this operation from a practical standpoint. It is thus surprising that, by treating the cells by the process of the invention, i.e. simultaneously with a water-soluble neutral salt and with a nonionic surfactant whose hydrophobic moiety comprises a substituted aromatic nucleus, the major part of the lysozyme associated with said cells is recovered, without having to destroy their structure. This shows that a major part of the lysozyme remaining associated with the yeast cells is not of intracellular nature, but that it is secreted into the periplasmic space thereof.

The salt to be used according to the invention is a water-soluble, neutral mineral salt. By way of example, there can be cited NaCl, KCl, $NaNO_3$, $KNO_3$, $Na_2SO_4$, etc. For both economical and biological reasons, NaCl is preferably used. The concentration at which the salt should be used is not critical; a concentration of at least 0.1 M will be used in order to obtain at best the desired result. In most cases, there is no advantage to use concentrations higher than 0.5 M.

The surfactant to be used according to the invention is selected from the nonionic ones. As opposed to anionic or cationic surfactants, the nonionic ones generally have the essential advantage not to induce in proteins any conformational modifications bringing on a reduction or even a loss of their biological activity. The Applicant has found that the nonionic surfactants which show a synergistic effect with water-soluble neutral mineral salts for the recovery of periplasmic proteins are those of the polyethoxylated alkylphenol type which are soluble in water and which have a HLB comprised between 8 and 15. As typical examples of such agents, there may be cited polyethoxylated octyl-, nonyl- and tributylphenols, particularly those commercially available under the trademarks Triton X-100, Nonidet P-40, Lutensol AP 8, Synperonic NP 10, Cemulsol OP 9, Sapogenat T-080, etc...

When selecting such a surfactant, it must be taken into account that the activity thereof is influenced not only by the substituted aromatic nucleus but also by the length of the ethoxylated chain. As a general rule, the surfactants having an ethoxylated chain such that their HLB is comprised between 8 and 15 will be selected. When the HLB is lower than 8, the solubility of the surfactant in water is generally insufficient. On the other hand, when the HLB is higher than 15, the synergistic effect claimed in this invention is too weak to be of any practical use. The concentration at which the surfactant is to be used is not very critical either. In most cases, a concentration comprised between 0.02 and 1% will be advantageously used.

According to this invention, the yeast cells to which is associated the polypeptide to be recovered are suspended in an aqueous medium comprising both the soluble neutral salt and the nonionic surfactant. The operating temperature is not critical. However, in order to minimize the denaturation of the polypeptides to be recovered, operating above room temperature will be avoided. On the other hand, it is necessary to allow a sufficient contact time between the cells and the medium. As a general rule, at temperatures close to room temperature, a contact time comprised between 30 and 120 minutes, more particularly between 45 and 90 minutes, is sufficient to obtain the expected result. The cells are then separated by centrifugation or by any other appropriate technique. They are preferably washed by resuspending them in the same medium or in any other aqueous medium, eventually in pure water. They can thereafter be separated again, then pressed or dried, i.e. undergo any useful operation for ensuring their valorisation, e.g. as protein source in animal feeding.

On the other hand, the proteins from the medium can be concentrated, separated and purified by any appropriate combination of techniques known in the art : lyophilisation, ultrafiltration, precipitation, chromatography, etc. As a matter of fact, the present invention is particularly useful to isolate a protein whose physico-chemical properties are such that it is difficult to separate it from a mixture with other proteins. Indeed, a protein which is associated either totally or partially to the periplasmic space can be separated easily, by simple centrifugation of the yeast cells, from other proteins present in solution in the culture medium. Further, by treating the yeasts isolated from their culture medium with a minimum of a medium according to the invention, the protein of interest is released in a relatively concentrated form, while avoiding its mixing with intracellular proteins.

The process of the invention can also be applied to the recovery of proteins secreted by yeasts immobilized by any means, by fixation on a solid support or inside a polymer gel, e.g. an alginate or an acrylamide gel. Other applications obvious for those skilled in the art are within the scope of the present invention. Similarly, although this invention is particularly appropriate for the recovery of heterologous proteins produced by yeasts of the S. cerevisiae species, as a result of a genetic manipulation, it can obviously be useful for the recovery of any polypeptide, whether heterologous or not, when said polypeptide is localized in the yeast periplasmic space, whatever may be the genus and the species of the yeast.

Example 1

Yeasts belonging to the species Saccharomyces cerevisiae, strain GRF 18 (auxotroph for leucine and histidine), and transformed by plasmid pLysΔ49 were grown at 28°C in minimal medium (glucose : 3%; yeast nitrogen base : 0.67%) supplemented with 0.002% histidine. Plasmid pLysΔ49 comprises the gene LEU2 conferring prototrophy for leucine to the transformed yeast ; it also contains the complete cDNA of chicken lysozyme (Belgian patent 901,223; strain deposited at the Centraal Bureau voor Schimmelcultures, Oosterstraat 1, Baarn, Netherlands on December 5, 1984 under n° CBS 7130).

When the culture reached the stationary phase, an aliquot part of 10 ml was centrifuged at 2500 g during 10 minutes. The cells were then resuspended in 3 ml of 0.1 M phosphate buffer pH 6.5 supplemented with 0.5 M NaCl and with a surfactant of the polyethoxylated p-octylphenol type (product sold by Rohm & Haas under the trade mark Triton X-100; said surfactant comprises generally 10 oxyethylene units per molecule) at a concentration of 0.05% by wt. By way of comparison, cells centrifuged from other aliquot parts of the culture were resuspended (1) in the phosphate buffer as such, (2) in the same buffer supplemented only with 0.5 M NaCl, and (3) in a buffer supplemented only with 0.05% Triton X-100. After 60 minutes incubation at 28°C in the various media, the cells were separated again by centrifugation, and lysozyme present in the supernatant was determined by the method of D. SHUGAR (Biochem. Biophys. Acta, 8, 302-309, 1952). In order to determine lysozyme still associated to the cells, these latter were resuspended in the same media and grinded by means of glass beads during 5 minutes in a Braun homogenizer. After separation of the cellular debris by centrifugation at 12,500 g, lysozyme present in the supernatant was determined as above.

The results obtained are shown in Table 1. It is seen that the addition of either 0.5 M NaCl or 0.05% Triton X-100 to the yeast suspension does not lead to any release of the lysozyme activity associated to the cells. This activity can only be detected in the homogenate obtained by grinding the cells in the presence of 0.5 M NaCl. By grinding in the absence of NaCl or in the presence of Triton X-100, the measured activity reaches only 20 to 30% of that observed in the presence of 0.5 M NaCl. In contrast, a significant synergistic effect on the release of lysozyme from the cells results from the joint presence of NaCl and Triton X-100 : in this case 88% of the activity measured in the homogenate is found in the first supernatant.

The fact that in the present example the amount of lysozyme released by application of the process of the invention is close to the total amount of lysozyme detectable by grinding the cells demonstrates that in the present case lysozyme is mainly localized in the yeast periplasmic space. However it may happen in other cases that a non-negligible part of lysozyme not yet secreted into the periplasmic space be still intracellular. In such cases, the yield in lysozyme recovered by the process of the invention should obviously be lower than that achieved in the present example.

Table 1

| Products added to 0.1 M phosphate buffer pH 6.5 | lysozyme activity (1) released | | |
|---|---|---|---|
| | without grinding the cells (A) | by grinding (B) | total (A + B) |
| - | 0 | 5.3 | 5.3 |
| 0.5 M NaCl | 0 | 25.6 | 25.6 |
| 0.05% Triton X-100 | 0 | 7.8 | 7.8 |
| 0.05% Triton X-100 + 0.5 M NaCl | 22.6 | 1.8 | 24.4 |

(1) Lysozyme activity is expressed in units per ml of initial culture. The measured results were corrected for the effect of the surfactant and/or the salt on the activity of lysozyme.

Example 2

The procedure described in Example 1 was repeated except that Triton X-100 was replaced by 1wt % Cemulsol OP-9 (polyethoxylated p-octylphenol having 9 oxyethylene units per molecule; product sold by Société Française d'Organo Synthèse, SFOS). The results obtained are shown in Table 2.

It can be seen that 21% of the lysozyme produced by the yeast is released, without grinding, by 0.5 M NaCl in the absence of surfactant. This may be explained by adsorption on the yeast wall of lysozyme already excreted into the medium. However, it is to be noted that with the process of the invention, an amount of lysozyme four times higher is obtained in the medium; this clearly shows, again, the synergistic effect of both constituents used in the process of the invention.

Table 2

| Products added to 0.1 M phosphate buffer pH 6.5 | Lysozyme activity (1) released | | |
|---|---|---|---|
| | without grinding the cells (A) | by grinding (B) | total (A + B) |
| - | 0 | 30.3 | 30.3 |
| 0.5 M NaCl | 22.2 | 83.3 | 105.5 |
| 1% Cemulsol OP-9 | 26.0 | 30.3 | 56.3 |
| 1% Cemulsol OP-9 + 0.5 M NaCl | 93.4 | 14.3 | 107.7 |

(1) see note (1) in Table 1

Example 3

This Example illustrates the effect of the surfactant concentration on the release of lysozyme localized in the yeast periplasmic space, in accordance with the process of the invention.

The procedure of Example 1 was repeated, but after centrifugation the cells were resuspended in the 0.1 M phosphate buffer pH 6.5 supplemented with 0.5 M NaCl and with various concentrations of Triton X-100. The results obtained after incubation of the cells as described in Example 1 are shown in Table 3.

For concentrations of Triton X-100 of 0.01% and lower, no release of lysozyme was observed. On the contrary, at a concentration of 0.05% an important release of the enzyme is observed which can still be improved by increasing the surfactant concentration beyond this value.

Table 3

| Products added to 0.1 H phosphate buffer pH 6.5 + 0.5 M NaCl | Lysozyme activity (1) released without grinding the cells |
|---|---|
| 0.001% Triton X-100 | 0 |
| 0.005% Triton X-100 | 0 |
| 0.01% Triton X-100 | 0 |
| 0.05% Triton X-100 | 24.4 |
| 0.1% Triton X-100 | 24.6 |
| 0.5% Triton X-100 | 29.6 |
| 1% Triton X-100 | 30.8 |

(1) see note (1) in Table 1

Example 4

This Example illustrates the effect of the concentration of the soluble salt on the release of lysozyme localized in the yeast periplasmic space, in accordance with the process of the invention.

The procedure of Example 1 was repeated but after centrifugation the cells were resuspended in the 0.1 M phosphate buffer pH 6.5 supplemented with 0.05% Triton X-100 and with various concentrations of NaCl. The results obtained after incubation of the cells as in Example 1 are shown in Table 4.

For a NaCl concentration of 0.1 M and lower, no release of lysozyme was observed. The whole of the lysozyme activity remains associated to the cells and can be partly measured by grinding. From a NaCl

6

concentration of 0.25 M, lysozyme activity was detected in the supernatant and reached a maxims for a concentration of 0.5 M.

Table 4

| Products added to 0.1 M phosphate buffer, pH 6.5 + 0.05% Triton X-100 | lysozyme activity (1) released | | |
|---|---|---|---|
| | without grinding the cells (A) | by grinding (B) | total (A + B) |
| 0.01 M NaCl | 0 | 4.9 | 4.9 |
| 0.05 M NaCl | 0 | 8.6 | 8.6 |
| 0.1 M NaCl | 0 | 11.7 | 11.7 |
| 0.25 M NaCl | 3.3 | 16.6 | 19.9 |
| 0.5 M NaCl | 22.6 | 1.8 | 24.4 |
| 1 M NaCl | 19.6 | 5.5 | 25.1 |

(1) See note (1) in Table 1

## Example 5

The procedure of Example 1 was repeated but by modifying the incubation time of the yeast cells in the phosphate buffer supplemented with 0.05% Triton X-100 and with 0.5 M NaCl. The results obtained are shown in Table 5.

It is seen that in order to release the lysozyme associated to the yeasts it is necessary to maintain the cells in contact with the medium for a sufficient time. After 30 minutes, an important release of the lysozyme is observed and can still be improved by further increasing the incubation time.

Table 5

| Incubation time (minutes) | Lysozmye activity (1) released | | |
|---|---|---|---|
| | without grinding the cells (A) | by grinding (B) | Total (A + B) |
| 0 | 0 | 33.5 | 33.5 |
| 10 | 5.4 | 24.7 | 30.1 |
| 20 | 9.7 | 21.6 | 31.3 |
| 30 | 12.6 | 20.1 | 32.7 |
| 60 | 15.0 | 17.9 | 32.9 |
| 120 | 16.1 | 15.4 | 31.5 |

(1) See note (1) in Table 1.

## Example 6

In this example, the effect of KCl is compared to that of NaCl. The procedure of Example 1 was repeated and the results obtained are shown in

It can be seen that the addition of 0.5 M KCl or NaCl to the yeast suspension medium does not lead to any release of the lysozyme activity associated to the cells. On the contrary, when the cells are suspended in a medium containing 0.5 M KCl and 0.05% Triton X-100, lysozyme is released in the same proportions as when NaCl is used as soluble salt.

Table 6

| Products added to the 0.1 M phosphate buffer pH 6.5 | Lysozyme activity (1) released without grinding the cells |
|---|---|
| 0.05% Triton X-100 | 0 |
| 0.5 M NaCl | 0 |
| 0.5 M KCl | 0 |
| 0.5 M NaCl + 0.05% Triton X-100 | 45.9 |
| 0.5 M KCl + 0.05% Triton X-100 | 43.3 |

(1) see note (1) Table 1

## Example 7

This Example illustrates the influence of various surfactants conforming to the general formula in accordance with the invention. The differences between all these surfactants reside in the alkyl substituents of the aromatic group and in the number n of oxyethylene units. By way of comparison other surfactants which do not correspond to the formula were tested.

The activity of these various surfactants was tested in the presence of 0.5 M NaCl under the conditions described in Example 1. The results obtained are shown in Table 7. It can be seen that from all tested surfactants, only those whose hydrophobic part has an aromatic ring substituted in accordance with the invention are active.

Table 7

| Products (a) added to the 0.1 M phosphate buffer pH 6.5 + 0.5 M NaCl | n | Lysozyme activity (b) released without grinding the cells |
|---|---|---|
| 0.1% Polyoxyethylene ether W-1 (Sigma Chemical) | | 0 (c) |
| 1% Sorbitan monolaurate (Radiamuls 2125, Oleofina) | | 0 (c) |
| 1% Sorbitan monooleate (Span 80, Atlas Chem. Ind.) | | 0 (c) |
| 1% Polyethoxylated sorbitan monooleate (Radiamuls 2137, Olefoine) (Tween 80, Atlas Chem Ind.) | (20) | 0 (c) |
| 1% Polyethoxylated sorbitan monostearate (Radiamuls 2147, Oleofina) | (20) | 0 (c) |
| 1% Polyethoxylated cetyl alcohol (Brij 58, Atlas Chem. Ind.) | (20) | 0 (c) |
| 0.2% Polyethoxylated p-octylphenol (Cemulsol OP-9, SFOS) | (9) | 81 |
| 0.05% Polyethoxylated p-octylphenol (Triton X-100, Rohm & Haas) | (10) | 88 |
| 0.2% Polyethoxylated p-nonylphenol (Synperonic NP 10, I.C.I.) | (10) | 61 |
| 0.2% Polyethoxylated tributylphenol (Sapogenat T-080, Hoechst) | (8) | 28 |

(a) In the case of polyethoxylated compounds according to the invention, the number n of oxyethylene units is given between parenthesis
(b) see note (1) in Table 1
(c) comparative examples.

## Example 8

This Example illustrates the influence of surfactants whose general formula and HLB ("Hydrophylic Lipophylic Balance") are in accordance with the present invention. By way of comparison, other surfactants were tested. These latter fulfil the same general formula but their HLB are outside the limits described in the present invention. The results obtained are shown in Table 8. It can be seen that in the latter case the release of lysozyme is weak or null.

Table 8

| Products (a) added to 0.1 M phosphate buffer pH 6.5 + 0.5 M NaCl Name (concentration : 0.2%) | (n) | Lysozyme activity (b) | |
|---|---|---|---|
| | | Theoretical HLB | released by system surfactant + NaCl without grinding of the cells |
| Polyethoxylated p-octylphenol (Renex 756, I.C.I.) | (6) | 11.3 | 77 |
| Polyethoxylated p-octylphenol (Cemulsol OP-9, SFOS) | (9) | 13.2 | 81 |
| Polyethoxylated p-octylphenol (Symperonic OP 14, I.C.I.) | (14) | 15.0 | 16 |
| Polyethoxylated p-octylphenol (Cemulsol OP 30, SFOS) | (30) | 17.3 | 0 (c) |
| Polyethoxylated p-nonylphenol (Lutensol AP 8, BASF) | (8) | 12.3 | 46 |
| Polyethoxylated p-nonylphenol (Synperonic OP 10, I.C.I.) | (10) | 13.4 | 41 |
| Polyethoxylated p-nonylphenol (Lutensol AP 14, BASF) | (14) | 14.8 | 4 |
| Polyethoxylated p-nonylphenol (Arkopal N 230, Hoechst) | (23) | 16.4 | 0 (c) |
| Polyethoxylated tributylphenol (Sapogenat T-040, Hoechst) | (4) | 8.1 | 18 |
| Polyethoxylated tributylphenol (Sapogenat T-080, Hoechst) | (8) | 11.4 | 32 |
| Polyethoxylated tributylphenol (Sapogenat T-180, Hoechst) | (18) | 15.0 | 0 (c) |
| Polyethoxylated tributylphenol (Sapogenat T-300, Hoechst) | (30) | 16.7 | 0 (c) |

(a) The number n of oxyethylene units per molecule is mentioned between parenthesis
(b) see note (1) in Table 1
(c) comparative examples

## Claims

1. Process for releasing into aqueous medium polypeptides soluble therein, produced by a yeast, and localized at least partially in the periplasmic space thereof, said process being characterized in that said polypeptides are released without cell lysis by treatment in an aqueous medium by a system comprising (i) a neutral and water-soluble mineral salt at a concentration of at least 0.1M , and (ii) a water-soluble, nonionic surfactant of the polyethoxylated alkylphenol type having a Hydrophilic Lipophilic Balance comprised between 8 and 15 at a concentration of at least 0.02%.

2. Process according to claim 1, wherein the yeast belongs to the <u>Saccharomyces</u> genus.

3. Process according to claim 2, wherein the yeast belongs to the species <u>Saccharomyces cerevisiae.</u>

4. Process according to claim 3, wherein the yeast belongs to the strain GRF 18.

5. Process according to any one of claims 1 to 4, wherein the yeast has been genetically manipulated to produce a polypeptide.

6. Process according to claim 5, wherein the polypeptide is an heterologous polypeptide.

7. Process according to claim 6, wherein the heterologous polypeptide is a lysozyme.

8. Process according to any one of Claims 1 to 7, wherein the nonionic surfactant is selected from the group comprising the polyethoxylated octyl- and nonylphenols having a Hydrophilic Lipophilic Balance comprised between 10 and 15.

9. Process according to any one of Claims 1 to 7, wherein the nonionic surfactant is a polyethoxylated tributylphenol having a Hydrophilic Lipophilic Balance comprised between 8 and 12.5.

## Revendications

1. Procédé assurant la libération dans un milieu aqueux de polypeptides solubles, produits par une levure et localisés au moins partiellement dans l'espace périplasmique de celle-ci, ce procédé étant caractérisé en ce que ces polypeptides sont libérés sans lyse des cellules par traitement, dans un milieu aqueux, avec un système comprenant:
    (i) un sel minéral neutre et soluble dans l'eau à une concentration d'au moins 0,1 M, et
    (ii) un agent tensioactif nonionique, soluble dans l'eau, du type alkylphénol polyéthoxylé ayant un HLB ("Hydrophilic Lipophilic Balance") compris entre 8 et 15, à une concentration d'au moins 0,02 %.

2. Procédé selon la revendication 1, caractérisé en ce que la levure appartient au genre <u>Saccharomyces.</u>

3. Procédé selon la revendication 2, caractérisé en ce que la levure appartient à l'espèce <u>Saccharomyces Cerevisiae.</u>

4. Procédé selon la revendication 3, caractérisé en ce que la levure appartient à la souche GRF18.

5. Procédé selon l'une quelconque des revendications 1 à 4, caractérisé en ce que la levure a été manipulée génétiquement pour produire un polypeptide.

6. Procédé selon la revendication 5, caractérisé en ce que le polypeptide est un polypeptide hétérologue.

7. Procédé selon la revendication 6, caractérisé en ce que le polypeptide hétérologue est un lysozyme.

8. Procédé selon l'une quelconque des revendications de 1 à 7, caractérisé en ce que l'agent tensioactif nonionique est choisi parmi le groupe comprenant les octyl- et nonylphénols polyéthoxylés ayant un HLB compris entre 10 et 15.

9. Procédé selon l'une quelconque des revendications de 1 à 7, caractérisé en ce que l'agent tensioactif nonionique est un tributylphénol polyéthoxylé dont le HLB est compris entre 8 et 12,5.

**Patentansprüche**

1. Verfahren zur Freisetzung von darin löslichen Polypeptiden in ein wäßriges Medium, wobei diese Polypeptide durch eine Hefe produziert wurden und wenigstens teilweise in deren periplasmatischem Raum lokalisiert sind und wobei das Verfahren dadurch gekennzeichnet ist, daß die Polypeptide ohne Zellauflösung in einem wäßrigen Medium durch eine Behandlung mit einem System freigesetzt werden, das umfaßt (i) ein neutrales und wasserlösliches Mineralsalz in einer Konzentration von wenigstens 0.1M, und (ii) ein wasserlösliches, nichtionisches Tensid vom Typ polyethoxylierter Alkylphenole mit einem Wert für das hydrophillipophile Gleichgewicht (HLB-Wert) zwischen 8 und 15 und in einer Konzentration von wenigstens 0.02%.

2. Verfahren nach Anspruch 1, worin die Hefe zur Gattung Saccharomyces gehört.

3. Verfahren nach Anspruch 2, worin die Hefe zur Art Saccharomyces cerevisiae gehört.

4. Verfahren nach Anspruch 3, worin die Hefe zum Stamm GRF 18 gehört.

5. Verfahren nach irgendeinem der Ansprüche 1 bis 4, worin die Hefe zur Erzeugung eines Polypeptides genetisch manipuliert wurde.

6. Verfahren nach Anspruch 5, worin das Polypeptid ein heterologes Polypeptid ist.

7. Verfahren nach Anspruch 6, worin das heterologe Polypeptid ein Lysozym ist.

8. Verfahren nach irgendeinem der Ansprüche 1 bis 7, worin das nichtionische Tensid aus der Gruppe ausgewählt ist, die die polyethoxylierten Octyl- und Nonylphenole mit einem HLB-Wert zwischen 10 und 15 umfaßt.

9. Verfahren nach irgendeinem der Ansprüche 1 bis 7, bei dem das nichtionische Tensid ein polyethoxyliertes Tributylphenol mit einem HLB-Wert zwischen 8 und 12,5 ist.